# EUROPEAN PATENT APPLICATION

(11) **EP 3 141 200 A1**
(43) Date of publication of application: **15.03.2017**
(21) Application number: 15192175.6
(22) Date of filing: 29.10.2015
(51) Int. Cl.: A61B 17/29

(54) **STEERABLE ENDOSCOPIC INSTRUMENT**

(30) Priority: 11.09.2015 TW 104130211
(71) Applicant: CoreBio Technologies Co., Ltd., Taoyuan City 335 (TW)
(72) Inventor: Hsu, Wen-Ming, 239 New Taipei City (TW); Li, Chih-Hung, 334 Taoyuan City (TW)
(74) Representative: Becker Kurig Straus

(57) **Abstract**

A steerable laparoscopic instrument (9) is provided. The steerable laparoscopic instrument (9) comprises a transmission part (91), a first disc (932), a second disc (922), a bending part (94), a third disc (921), a working part (95), and a handle part (93). The steerable laparoscopic instrument (9) provides surgical operation with at least two directional controls, is easily disassembled and reassembled, can be operated by a single hand, and is easily to be cleaned and sterilized.

## Description

### FIELD OF THE INVENTION

The present invention relates to a laparoscopic instrument, and more particularly to a steerable laparoscopic instrument.

### BACKGROUND OF THE INVENTION

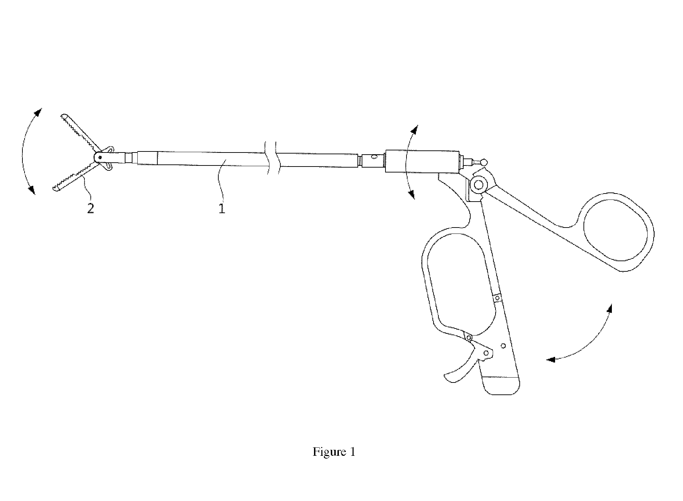

As shown in **Fig. 1****,** a conventional laparoscopic instrument typically has only a single directional control, i.e., the 360 degree rotational control of tube **1** allowing the working part 2 to work at various angles. The working part **2** may be scissors, pliers, or the like. However, to adjust the working part 2 of such instrument at a proper orientation during minimally invasive surgery would be difficult due to limited operation space.

Therefore, it is urgently desired in this field to develop an improved laparoscopic instrument which provides multiple directional controls for the surgeon to have maximal degree of freedom in performing minimally invasive surgery.

### SUMMARY OF THE INVENTION

Accordingly, the present invention provides a steerable laparoscopic instrument, which has at least two directional controls. The steerable laparoscopic instrument comprises a transmission part, a first disc, a second disc, a bending part, a third disc, a working part, and a handle part.

The transmission part has a first end and a second end, and comprises an inner tube and an outer tube, both of which extend along a first axis.

The first disc is disposed at the first end of the transmission part. The first disc is coupled with the outer tube and is used to control the rotation of the outer tube on the first axis.

The second disc is also disposed at the first end of the transmission part. The second disc is coupled with the inner tube and is used to control the movement of the inner tube along the first axis.

The bending part is pivotally connected to the second end of the transmission part. Further, the bending part has a sliding linkage pivotally connected thereto. The sliding linkage is coupled with the inner tube.

The third disc is disposed at the first end of the transmission part. The third disc comprises a center transmission rod coupled therewith. The center transmission rod extends along the first axis.

The working part is connected to the bending part. The working part extends along a second axis and has a connecting member, a third end, a fourth end and a plier-like member. The connecting member is disposed at the third end, and the plier-like member is disposed at the fourth end. Further, the working part is coupled with the third disc through the center transmission rod.

The handle part is disposed at a side of the first end of the transmission part. The handle part comprises a control member and a handle. The control member is coupled with the center transmission rod and is used to control the movement of the center transmission rod along the first axis.

According to the present invention, it is preferable that at least a section of the center transmission rod is of superelastic material, wherein the section is a section of the center transmission rod that has a position corresponding to the bending part.

In one embodiment of the present invention, the superelastic material is a shape memory alloy.

According to the present invention, the movement of the sliding linkage changes the angle between the first and second axes by moving back and forth along the first axis.

In one embodiment of the present invention, the connecting member connects to the bending part through a buckle.

In certain embodiments of the present invention, the handle part further comprises a locking member. The locking member is disposed at a side of the handle part and is used to lock the control member to the handle in tooth-type, cam-type or spring-type.

In certain embodiments of the present invention, the opening and closing of the plier-like member is controlled by a central screw driven by an inner torsion spring or an outer torsion spring.

According to one embodiment of the present invention, the steerable laparoscopic instrument further comprises a housing for accommodating the first, second and third discs.

### BRIEF DESCRIPTION OF THE DRAWINGS

The foregoing summary, as well as the following detailed description of the invention, will be better understood when read in conjunction with the appended drawing. In the drawings:
Figure 1 is a schematic view of a conventional laparoscopic instrument.
Figure 2 is a perspective view of a steerable laparoscopic instrument of the present invention.
Figure 3 shows the relationship between the working part and the bending part of a steerable laparoscopic instrument of the present invention.
Figure 4 illustrates the locking member of the handle part of a steerable laparoscopic instrument of the present invention.

### DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereof known to those skilled in the art.

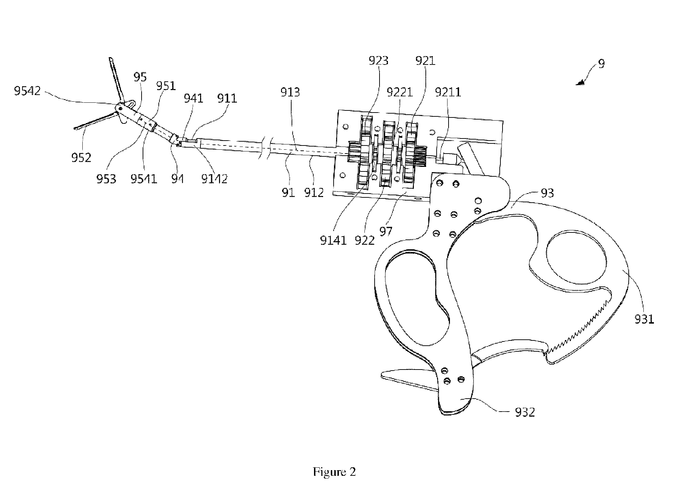

Referring to **Fig. 2****,** shown is an embodiment of the steerable laparoscopic instrument according to the present invention. A steerable laparoscopic instrument **9** comprises a transmission part **91,** a first disc **923,** a second disc **922,** a bending part **94,** a third disc **921,** a working part **95,** a handle part **93,** and a housing **97.**

The transmission part **91** extends along a first axis **913,** and includes an outer tube **912** and an inner tube **911** movably disposed inside the outer tube **912.** Both of the inner and outer tubes **911** and **912** extend along the first axis **913.** The transmission part **91** has a first end **9141** and a second end **9142.**

The first disc **923** is disposed at the first end **9141** of the transmission part **91.** The first disc **923** is coupled with the outer tube **912** and is used to control (or manipulate) the rotation of the outer tube **912** on the first axis **913.** In other words, the first disc **923** is coupled with the outer tube **912** in a manner such that the rotation of the first disc **923** on the first axis **913** drives the outer tube **912** to rotate on the first axis **913** correspondingly.

The second disc **922** is also disposed at the first end **9141** of the transmission part **91.** As shown in **Fig. 2****,** the second disc **922** may be disposed adjacent the first disc **923.** In addition, the second disc **922** is coupled with the inner tube **911** in a manner such that the rotation of the second disc **922** on the first axis **913** drives the inner tube **911** to move along the first axis **913** correspondingly. For example, when the second disc **922** is rotated clockwise on the first axis **913,** the inner tube **911** moves correspondingly toward the direction of the second end **9142** of the transmission part **91** (to the left in **Fig. 2**) along the first axis **913;** and when the second disc **922** is rotated counterclockwise on the first axis **913,** the inner tube **911** moves correspondingly toward the direction of the first end **9141** of the transmission part **91** (to the right in **Fig. 2****)** along the first axis **913.** In the present embodiment, the second disc **922** is coupled with the inner tube **911** through a screw nut **9221** which is co-axial with the first axis **913.**

In addition, the bending part **94** is pivotally connected to the second end **9142** of the transmission part **91.** Specifically, the bending part **94** is pivotally connected to the outer tube **912** of the transmission part **91.** A sliding linkage **941** is pivotally connected to the bending part **94.** The sliding linkage **941** is coupled with the inner tube **911,** such that the movement of the inner tube **911** along the first axis **913** drives the sliding linkage to move along the first axis **913** correspondingly. Accordingly, when rotating the second disc **922** and the screw nut **9221,** the movement of the sliding linkage causes the rotation of the bending part **94** with respect to the pivot point where the bending part **94** is pivoted to the second end **9142** of the transmission part **91.**

The third disc **921** is disposed at the first end **9141** of the transmission part **91.** A center transmission rod **9211** extends along the first axis **913** is coupled with the third disc **921.** Preferably, the center transmission rod **9211** is coupled with the third disc **921** in a manner such that the rotation of the third disc **921** on the first axis **913** drives the center transmission rod 9211 to rotate correspondingly on the first axis **913.**

The working part **95** is connected to the bending part **94.** The working part **95** extends along a second axis **953** and has a connecting member **951,** a third end **9541,** a fourth end **9542** and a plier-like member **952.** The connecting member **951** is disposed at the third end **9541** and the plier-like member **952** is disposed at the fourth end **9542.** The working part **95** is coupled with the third disc **921** through the center transmission rod **9211** in a manner such that the rotation of the third disc **921** on the first axis **913** drives the rotation of the working part **95** on the first axis **913**correspondingly. Put differently, the center transmission rod **9211** run through the interior of the transmission part **91,** the bending part **94** and the working part **95,** and one end of the center transmission rod **9211** is connected to the working part 95 while the other end of the center transmission rod **9211** is connected to the third disc **921.**

Further, the handle part **93** is disposed at a side of the first end **9141** of the transmission part **91.** The handle part **93** includes a control member **931** and a handle **932.** The control member **931** is coupled with the center transmission rod **9211** and is used to control the movement of the center transmission rod **9211** along the first axis **913.** The movement of the center transmission rod **9211** along the first axis **913** is for the control of the opening and closing of the plier-like member **952** of the working part **95.**

The steerable laparoscopic instrument **9** may further comprise a housing **97** for accommodating the first disc **923,** the second disc **922** and the third disc **921.** The housing **97** may be connected to the handle **932.**

Accordingly, the outer tube **923** (along with the working part **95**) is up to 360 degree rotatable on the first axis **913** by rotating the first disc **923;** the transmission part **94** (along with the working part **95**) is steerable with respect to the first axis **913** by rotating the second disc **923** (the angle between the first and second axes **913** and **953** is changed accordingly); and the working part **95** (along with the plier-like member **952**) is up to 360 degree rotatable on the second axis **953,** independently from the rotation of the outer tube **923,** by rotating the third disc **921.**

With reference to **Fig. 2** again, in accordance with one preferred embodiment of the present invention, a section of the center transmission rod **9211** having a position corresponding to the bending part **94** is of superelastic (or termed as "pseudoelastic") material. Alternatively, the whole center transmission rod **9211** may be made of a superelastic material. The superelastic material includes but is not limited to a shape memory alloy, for example, a nickel-titanium alloy. In one embodiment of the present invention, the nickel-titanium alloy used has the following properties: (I) Mechanical properties: (a) tensile strength: 830∼1170 MPa, (b) yield strength: 200 MPa, (c) Young's modulus: 70 GPa, (d) Poisson's ratio: 0.33, and (e) hardness: 80 RB; and (II) Shape memory properties: (a) transformation temperature: - 20∼+150°C; (b) shape memory strength: 550MPa; and (c) deformation amount: 6%.

Referring to **Fig. 2****,** in accordance with one embodiment of the present invention, the movement of the sliding linkage **941** moves the working part **95** in link-type and changes the angle formed between the first and second axes **913** and **953,** wherein said angle may be greater than 90 degrees.

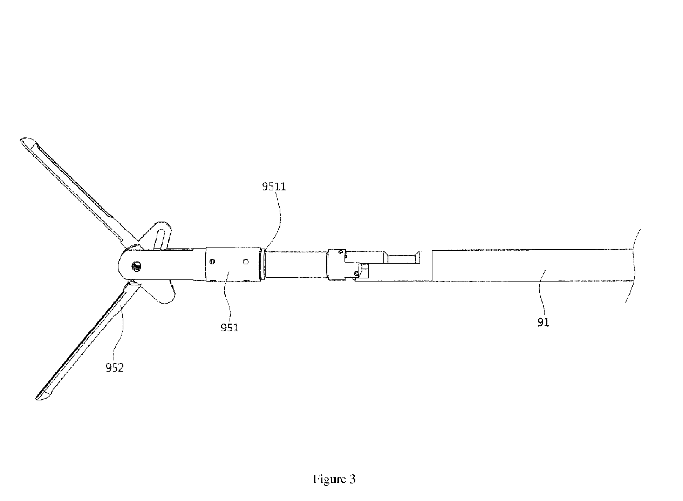

With reference to **Fig. 3****,** in accordance with one embodiment of the present invention, the connecting member **951** removably connects to the bending part **94** through a buckle **9511.** The plier-like member **952** may be pliers, scissors, claws pliers or a needle holder, but is not limited thereto.

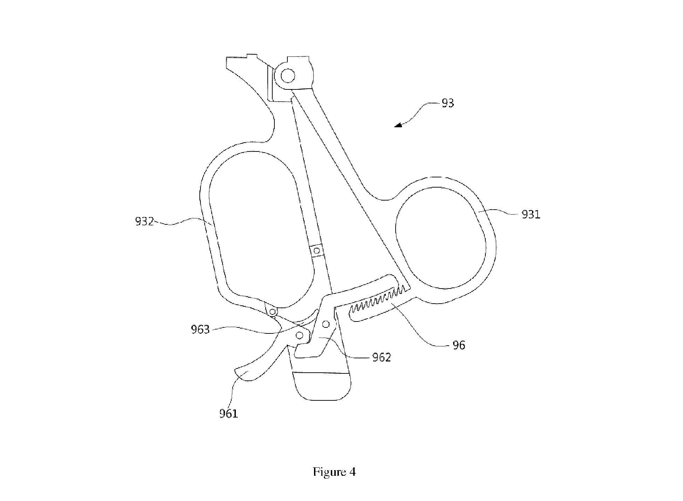

Referring to **Fig. 4****,** the handle part **93** further comprises a locking member **96.** The locking member **96** is disposed at a side of the handle part **93** and is used to lock the control member **931** to the handle **932** in tooth-type, cam-type or spring-type. In one preferred embodiment, the locking member **96** comprises a switch **961,** a pawl **962,** and a spring sheet **963.** In operation, when the switch **961** is not pressed, the control member **931** can only be moved in one direction and the handle part **93** is in a locked state; for a semi-unlocked state, the pawl **962** is moved away from the locking member **96** when the switch **961** is pressed, and the control member **931** can be freely moved in both directions to control the opening and closing of the plier-like member **952** with the switch **961** being continuously pressed; and when the switch **961** is pushed upward, the pawl **962** would not bounce back by spring sheet **963** since the locking member **96** is in an irreversible position, and thus the handle part **93** is in an unlocked state.

In one embodiment of the present invention, the opening and closing of the plier-like member **952** is controlled by a central screw driven by an inner torsion spring or an outer torsion spring.

In the development of the present invention, considering the relevance between manipulation and transmission under displacement controlled design, the durability under elastic deformation, wear resistant shape design, assembly processing costs and mechanism durability, the difficulties on the small mechanism space, space limitation and durability of the transmission transition and whether a complicated mechanism can be easily disassembled and reassembled were overcome, and the following advantages were achieved: small limitation on the steering angle, multiple rotation/steering functions, convenience on disassembling, cleaning and replaceability, and high durability.

It is believed that a person of ordinary knowledge in the art where the present invention belongs can utilize the present invention to its broadest scope based on the descriptions herein with no need of further illustration. Therefore, the descriptions and claims as provided should be understood as of demonstrative purpose instead of limitative in any way to the scope of the present invention.

## Claims

1. A steerable laparoscopic instrument, comprising:
a transmission part extending along a first axis, including an inner tube and an outer tube extending along said first axis, the transmission part having a first end and a second end;
a first disc disposed at the first end of the transmission part, coupled with the outer tube and used to control the rotation of the outer tube on the first axis;
a second disc disposed at the first end of the transmission part, coupled with the inner tube and used to control the movement of the inner tube along the first axis;
a bending part pivotally connected to the second end of the transmission part, having a sliding linkage pivotally connected thereto, the sliding linkage being coupled with the inner tube;
a third disc disposed at the first end of the transmission part, including a center transmission rod extending along the first axis coupled therewith;
a working part connected to the bending part, which extends along a second axis and has a connecting member, a third end, a fourth end and a plier-like member, the connecting member being disposed at the third end and the plier-like member being disposed at the fourth end, the working part being coupled with the third disc through the center transmission rod; and
a handle part disposed at a side of the first end of the transmission part, including a control member and a handle, the control member being coupled with the center transmission rod and used to control the movement of the center transmission rod along the first axis.

2. The steerable laparoscopic instrument of claim 1, wherein a section of the center transmission rod having a position corresponding to the bending part is of superelastic material.

3. The steerable laparoscopic instrument of claim 2, wherein the superelastic material is a shape memory alloy.

4. The steerable laparoscopic instrument of claim 1, wherein the movement of the center transmission rod along the first axis is used to control the opening and closing of the plier-like member of the working part

5. The steerable laparoscopic instrument of claim 1, wherein the movement of the sliding linkage moves the working part in link-type and changes the angle formed between the first and second axes.

6. The steerable laparoscopic instrument of claim 1, wherein the connecting member connects to the bending part through a buckle.

7. The steerable laparoscopic instrument of claim 1, wherein the handle part further includes a locking member disposed at a side of the handle part, the locking member being used to lock the control member to the handle in tooth-type, cam-type or spring-type.

8. The steerable laparoscopic instrument of claim 1, wherein the opening and closing of the plier-like member is controlled by a central screw driven by an inner torsion spring or an outer torsion spring.

9. The steerable laparoscopic instrument of claim 1, further comprising a housing for accommodating the first, second and third discs.
